# NEUE EUROPÄISCHE PATENTSCHRIFT

(11) **EP 2 992 093 B2**
(45) Veröffentlichungstag und Bekanntmachung des Hinweises auf die Entscheidung über den Einspruch: **13.11.2024**
(45) Hinweis auf die Patenterteilung: 18.03.2020
(21) Anmeldenummer: 14720548.8
(22) Anmeldetag: 23.04.2014
(51) Int. Cl.: C12N 9/54, C11D 3/386

(54) **REINIGUNGSMITTEL ENTHALTEND PROTEASEN**
CLEANING AGENT CONTAINING PROTEASES
PRODUIT DÉTERGENT CONTENANT DES PROTÉASES

(30) Priorität: 30.04.2013 DE 102013207933
(43) Veröffentlichungstag der Anmeldung: 09.03.2016
(73) Patentinhaber: Henkel AG & Co. KGaA, 40589 Düsseldorf (DE)
(72) Erfinder: EITING, Thomas, 40589 Düsseldorf (DE); MUßMANN, Nina, 47877 Willich (DE); BASTIGKEIT, Thorsten, 42279 Wuppertal (DE); WRUBBEL, Noelle, 40591 Düsseldorf (DE)
(86) Internationale Anmeldenummer: PCT/EP2014/058176
(87) Internationale Veröffentlichungsnummer: WO 2014/177430

(56) Entgegenhaltungen:
- EP-A1- 2 216 393
- EP-A1- 2 551 335
- WO-A1-2011/072117
- WO-A2-2009/021867
- US-A1- 2012 172 280

### SEQUENCE LISTING

<110> Henkel AG & Co. KGaA
<120> Reinigungsmittel enthaltend Proteasen
<130> PT031731_PCT
<150> DE102013207933.8
   <151> 2013-04-30
<160> 6
<170> PatentIn version 3.5
<210> 1
   <211> 269
   <212> PRT
   <213> Bacillus lentus
<400> 1
   Ala Gln Ser Val Pro Trp Gly Ile Ser Arg Val Gln Ala Pro Ala Ala
   1               5                   10                  15
   His Asn Arg Gly Leu Thr Gly Ser Gly Val Lys Val Ala Val Leu Asp
               20                  25                  30
   Thr Gly Ile Ser Thr His Pro Asp Leu Asn Ile Arg Gly Gly Ala Ser
           35                  40                  45
   Phe Val Pro Gly Glu Pro Ser Thr Gln Asp Gly Asn Gly His Gly Thr
       50                  55                  60
   His Val Ala Gly Thr Ile Ala Ala Leu Asn Asn Ser Ile Gly Val Leu
   65                  70                  75                  80
   Gly Val Ala Pro Ser Ala Glu Leu Tyr Ala Val Lys Val Leu Gly Ala
                   85                  90                  95
   Ser Gly Ser Gly Ser Val Ser Ser Ile Ala Gln Gly Leu Glu Trp Ala
               100                 105                 110
   Gly Asn Asn Gly Met His Val Ala Asn Leu Ser Leu Gly Ser Pro Ser
           115                 120                 125
   Pro Ser Ala Thr Leu Glu Gln Ala Val Asn Ser Ala Thr Ser Arg Gly
       130                 135                 140
   Val Leu Val Val Ala Ala Ser Gly Asn Ser Gly Ala Gly Ser Ile Ser
   145                 150                 155                 160
   Tyr Pro Ala Arg Tyr Ala Asn Ala Met Ala Val Gly Ala Thr Asp Gln
                   165                 170                 175
   Asn Asn Asn Arg Ala Ser Phe Ser Gln Tyr Gly Ala Gly Leu Asp Ile
               180                 185                 190
   Val Ala Pro Gly Val Asn Val Gln Ser Thr Tyr Pro Gly Ser Thr Tyr
           195                 200                 205
   Ala Ser Leu Asn Gly Thr Ser Met Ala Thr Pro His Val Ala Gly Ala
       210                 215                 220
   Ala Ala Leu Val Lys Gln Lys Asn Pro Ser Trp Ser Asn Val Gln Ile
   225                 230                 235                 240
   Arg Asn His Leu Lys Asn Thr Ala Thr Ser Leu Gly Ser Thr Asn Leu
                   245                 250                 255
   Tyr Gly Ser Gly Leu Val Asn Ala Glu Ala Ala Thr Arg
               260                 265
<210> 2
   <211> 269
   <212> PRT
   <213> Bacillus lentus
<400> 2
   Ala Gln Ser Val Pro Trp Gly Ile Ser Arg Val Gln Ala Pro Ala Ala
   1               5                   10                  15
   His Asn Arg Gly Leu Thr Gly Ser Gly Val Lys Val Ala Val Leu Asp
               20                  25                  30
   Thr Gly Ile Ser Thr His Pro Asp Leu Asn Ile Arg Gly Gly Ala Ser
           35                  40                  45
   Phe Val Pro Gly Glu Pro Ser Thr Gln Asp Gly Asn Gly His Gly Thr
       50                  55                  60
   His Val Ala Gly Thr Ile Ala Ala Leu Asn Asn Ser Ile Gly Val Leu
   65                  70                  75                  80
   Gly Val Ala Pro Ser Ala Glu Leu Tyr Ala Val Lys Val Leu Gly Ala
                   85                  90                  95
   Asp Gly Arg Gly Ala Ile Ser Ser Ile Ala Gln Gly Leu Glu Trp Ala
               100                 105                 110
   Gly Asn Asn Gly Met His Val Ala Asn Leu Ser Leu Gly Ser Pro Ser
                   115                 120                 125
   Pro Ser Ala Thr Leu Glu Gln Ala Val Asn Ser Ala Thr Ser Arg Gly
       130                 135                 140
   Val Leu Val Val Ala Ala Ser Gly Asn Ser Gly Ala Ser Ser Ile Ser
   145                 150                 155                 160
   Tyr Pro Ala Arg Tyr Ala Asn Ala Met Ala Val Gly Ala Thr Asp Gln
                   165                 170                 175
   Asn Asn Asn Arg Ala Ser Phe Ser Gln Tyr Gly Ala Gly Leu Asp Ile
               180                 185                 190
   Val Ala Pro Gly Val Asn Val Gln Ser Thr Tyr Pro Gly Ser Thr Tyr
           195                 200                 205
   Ala Ser Leu Asn Gly Thr Ser Met Ala Thr Pro His Val Ala Gly Ala
       210                 215                 220
   Ala Ala Leu Val Lys Gln Lys Asn Pro Ser Trp Ser Asn Val Gln Ile
   225                 230                 235                 240
   Arg Asn His Leu Lys Asn Thr Ala Thr Ser Leu Gly Ser Thr Asn Leu
                   245                 250                 255
   Tyr Gly Ser Gly Leu Val Asn Ala Glu Ala Ala Thr Arg
               260                 265
<210> 3
   <211> 269
   <212> PRT
   <213> Artificial
<220>
   <223> Optimized B. lentus subtilisin 309 variant
<400> 3
   Ala Gln Ser Val Pro Trp Gly Ile Arg Arg Val Gln Ala Pro Thr Ala
   1               5                   10                  15
   His Asn Arg Gly Leu Thr Gly Ser Gly Val Lys Val Ala Val Leu Asp
               20                  25                  30
   Thr Gly Ile Ser Thr His Pro Asp Leu Asn Ile Arg Gly Gly Ala Ser
           35                  40                  45
   Phe Val Pro Gly Glu Pro Ser Thr Gln Asp Gly Asn Gly His Gly Thr
       50                  55                  60
   His Ala Ala Gly Thr Ile Ala Ala Leu Asn Asn Ser Ile Gly Val Leu
   65                  70                  75                  80
   Gly Val Ala Pro Ser Ala Glu Leu Tyr Ala Val Lys Val Leu Gly Ala
                   85                  90                  95
   Ser Gly Ser Gly Ser Val Ser Ser Ile Ala Gln Gly Leu Glu Trp Ala
               100                 105                 110
   Gly Asn Asn Gly Met His Val Ala Asn Leu Ser Leu Gly Ser Pro Ser
           115                 120                 125
   Pro Ser Ala Thr Leu Glu Gln Ala Val Asn Ser Ala Thr Ser Arg Gly
       130                 135                 140
   Val Leu Val Val Ala Ala Ser Gly Asn Ser Gly Ala Gly Ser Ile Ser
   145                 150                 155                 160
   Tyr Pro Ala Arg Tyr Ala Asn Ala Met Ala Val Gly Ala Thr Asp Gln
                   165                 170                 175
   Asn Asn Asn Arg Ala Ser Phe Ser Gln Tyr Gly Ala Gly Leu Asp Ile
               180                 185                 190
   Val Ala Pro Gly Val Asn Val Gln Ser Thr Tyr Pro Gly Ser Thr Tyr
           195                 200                 205
   Ala Ser Leu Asp Gly Thr Ser Met Ala Thr Pro His Val Ala Gly Ala
       210                 215                 220
   Ala Ala Leu Val Lys Gln Lys Asn Pro Ser Trp Ser Asn Val Arg Ile
   225                 230                 235                 240
   Arg Asn His Leu Lys Asn Thr Ala Thr Ser Leu Gly Ser Thr Asn Leu
                   245                 250                 255
   Tyr Gly Ser Gly Leu Val Asn Ala Glu Ala Ala Thr Arg
               260                 265
<210> 4
   <211> 269
   <212> PRT
   <213> Artificial
<220>
   <223> Optimized B. lentus alkaline protease variant
<400> 4
   Ala Gln Ser Val Pro Trp Gly Ile Ser Arg Val Gln Ala Pro Ala Ala
   1               5                   10                  15
   His Asn Arg Gly Leu Thr Gly Ser Gly Val Lys Val Ala Val Leu Asp
               20                  25                  30
   Thr Gly Ile Ser Thr His Pro Asp Leu Asn Ile Arg Gly Gly Ala Ser
           35                  40                  45
   Phe Val Pro Gly Glu Pro Ser Thr Gln Asp Gly Asn Gly His Gly Thr
       50                  55                  60
   His Val Ala Gly Thr Ile Ala Ala Leu Asn Asn Ser Ile Gly Val Leu
   65                  70                  75                  80
   Gly Val Ala Pro Ser Ala Glu Leu Tyr Ala Val Lys Val Leu Gly Ala
                   85                  90                  95
   Asp Gly Glu Gly Ala Ile Ser Ser Ile Ala Gln Gly Leu Glu Trp Ala
               100                 105                 110
   Gly Asn Asn Gly Met His Val Ala Asn Leu Ser Leu Gly Ser Pro Ser
           115                 120                 125
   Pro Ser Ala Thr Leu Glu Gln Ala Val Asn Ser Ala Thr Ser Arg Gly
       130                 135                 140
   Val Leu Val Val Ala Ala Ser Gly Asn Ser Gly Ala Ser Ser Ile Ser
   145                 150                 155                 160
   Tyr Pro Ala Arg Tyr Ala Asn Ala Met Ala Val Gly Ala Thr Asp Gln
                   165                 170                 175
   Asn Asn Asn Arg Ala Ser Phe Ser Gln Tyr Gly Ala Gly Leu Asp Ile
               180                 185                 190
   Val Ala Pro Gly Val Asn Val Gln Ser Thr Tyr Pro Gly Ser Thr Tyr
           195                 200                 205
   Ala Ser Leu Asn Gly Thr Ser Met Ala Thr Pro His Val Ala Gly Ala
       210                 215                 220
   Ala Ala Leu Val Lys Gln Lys Asn Pro Ser Trp Ser Asn Val Gln Ile
   225                 230                 235                 240
   Arg Asn His Leu Lys Asn Thr Ala Thr Ser Leu Gly Ser Thr Asn Leu
                   245                 250                 255
   Tyr Gly Ser Gly Leu Val Asn Ala Glu Ala Ala Thr Arg
   260 265
<210> 5
   <211> 269
   <212> PRT
   <213> Artificial
<220>
   <223> Optimized B. lentus alkaline protease variant
<400> 5
   Ala Gln Thr Ile Pro Trp Gly Ile Ser Arg Val Gln Ala Pro Ala Ala
   1 5 10 15
   His Asn Arg Gly Leu Thr Gly Ser Gly Val Lys Val Ala Val Leu Asp
   20 25 30
   Thr Gly Ile Ser Thr His Pro Asp Leu Asn Ile Arg Gly Gly Ala Ser
   35 40 45
   Phe Val Pro Gly Glu Pro Ser Thr Gln Asp Gly Asn Gly His Gly Thr
   50 55 60
   His Val Ala Gly Thr Ile Ala Ala Leu Asn Asn Ser Ile Gly Val Leu
   65 70 75 80
   Gly Val Ala Pro Ser Ala Glu Leu Tyr Ala Val Lys Val Leu Gly Ala
   85 90 95
   Asp Gly Glu Gly Ala Ile Ser Ser Ile Ala Gln Gly Leu Glu Trp Ala
   100 105 110
   Gly Asn Asn Gly Met His Val Ala Asn Leu Ser Leu Gly Ser Pro Ser
   115 120 125
   Pro Ser Ala Thr Leu Glu Gln Ala Val Asn Ser Ala Thr Ser Arg Gly
   130 135 140
   Val Leu Val Val Ala Ala Ser Gly Asn Ser Gly Ala Ser Ser Ile Ser
   145 150 155 160
   Tyr Pro Ala Arg Tyr Ala Asn Ala Met Ala Val Gly Ala Thr Asp Gln
   165 170 175
   Asn Asn Asn Arg Ala Ser Phe Ser Gln Tyr Gly Ala Gly Leu Asp Ile
   180 185 190
   Val Ala Pro Gly Val Asn Ile Gln Ser Thr Tyr Pro Gly Ser Thr Tyr
           195                 200                 205
   Ala Ser Leu Asn Gly Thr Ser Met Ala Thr Pro His Val Ala Gly Ala
       210                 215                 220
   Ala Ala Leu Val Lys Gln Lys Asn Pro Ser Trp Ser Asn Val Gln Ile
   225                 230                 235                 240
   Arg Asn His Leu Lys Asn Thr Ala Thr Ser Leu Gly Ser Thr Asn Leu
                   245                 250                 255
   Tyr Gly Ser Gly Leu Val Asn Ala Glu Ala Ala Thr Arg
               260                 265
<210> 6
   <211> 270
   <212> PRT
   <213> Artificial
<220>
   <223> Optimized B. lentus subtilisin 309 variant
<400> 6
   Ala Gln Ser Val Pro Trp Gly Ile Ser Arg Val Gln Ala Pro Ala Ala
   1               5                   10                  15
   His Asn Arg Gly Leu Thr Gly Ser Gly Val Lys Val Ala Val Leu Asp
               20                  25                  30
   Thr Gly Ile Ser Thr His Pro Asp Leu Asn Ile Arg Gly Gly Ala Ser
           35                  40                  45
   Phe Val Pro Gly Glu Pro Ser Thr Gln Asp Gly Asn Gly His Gly Thr
       50                  55                  60
   His Val Ala Gly Thr Ile Ala Ala Leu Asn Asn Ser Ile Gly Val Leu
   65                  70                  75                  80
   Gly Val Ala Pro Ser Ala Glu Leu Tyr Ala Val Lys Val Leu Gly Ala
                   85                  90                  95
   Ser Gly Ala Asp Gly Ser Val Ser Ser Ile Ala Gln Gly Leu Glu Trp
               100                 105                 110
   Ala Gly Asn Asn Gly Met His Val Ala Asn Leu Ser Leu Gly Ser Pro
           115                 120                 125
   Ser Pro Ser Ala Thr Leu Glu Gln Ala Val Asn Ser Ala Thr Ser Arg
       130                 135                 140
   Gly Val Leu Val Val Ala Ala Ser Gly Asn Ser Gly Ala Gly Ser Ile
   145                 150                 155                 160
   Ser Tyr Pro Ala Arg Tyr Ala Asn Ala Met Ala Val Gly Ala Thr Asp
                   165                 170                 175
   Gln Asn Asn Asn Arg Ala Ser Phe Ser Gln Tyr Gly Ala Gly Leu Asp
               180                 185                 190
   Ile Val Ala Pro Gly Val Asn Val Gln Ser Thr Tyr Pro Gly Ser Thr
           195                 200                 205
   Tyr Ala Ser Leu Asn Gly Thr Ser Met Ala Thr Pro His Val Ala Gly
       210                 215                 220
   Ala Ala Ala Leu Val Lys Gln Lys Asn Pro Ser Trp Ser Asn Val Gln
   225                 230                 235                 240
   Ile Arg Asn His Leu Lys Asn Thr Ala Thr Ser Leu Gly Ser Thr Asn
                   245                 250                 255
   Leu Tyr Gly Ser Gly Leu Val Asn Ala Glu Ala Ala Thr Arg
               260                 265                 270

## Beschreibung

Die vorliegende Anmeldung richtet sich auf ein Geschirrspülmittel, vorzugsweise ein maschinelles Geschirrspülmittel, das mindestens zwei Proteasen enthält, sowie die Verwendung eines solchen Mittels.

Das wichtigste Kriterium beim Reinigen von Textilien, harten Oberflächen, wie insbesondere beim Geschirrspülen, insbesondere beim maschinellen Geschirrspülen, ist die Reinigungsleistung an verschiedensten Anschmutzungen, welche insbesondere in Form von Lebensmittelresten eingebracht werden. Auch wenn die Reinigungsleistung der heutzutage eingesetzten Geschirrspülmittel generell hoch ist, stellt sich, auch aufgrund des generellen Trends beim maschinellen Geschirrspülen vermehrt Niedrigtemperatur-Programme zu verwenden, allerdings das Problem, dass viele der üblichen maschinellen Geschirrspülmittel bei hartnäckigen, angebrannten Anschmutzungen eine unzureichende Reinigungsleistung aufweisen. Eine solche unzureichende Reinigungsleistung und die damit einhergehende unzureichende Reinigung des Geschirrs führen beim Verbraucher zu Unzufriedenheit und dazu, dass hartnäckige Anschmutzungen vom Verbraucher vorbehandelt werden, was wiederum den Wasser- und Energieverbrauch erhöht. Aus z.B. EP2216393A1, WO2011072117A1, EP2551335A1 und WO2009021867A2 sind Reinigungsmittelzusammensetzung mit Enzymen, insbesondere z.B. einer oder zwei Proteasen bekannt, wobei der Einsatz von Enzymen zur Entfernung von speziellen Flecken dient. Es besteht dennoch ein allgemeiner Bedarf nach weiter verbesserten maschinellen Geschirrspülmitteln, die auch an hartnäckigen, insbesondere angebrannten Anschmutzungen noch eine gute Reinigungsleistung aufweisen, ohne dabei die bestehende gute Reinigungsleistung an anderen Anschmutzungen zu verringern.

Die Aufgabe der vorliegenden Erfindung bestand daher darin, ein Geschirrspülmittel, vorzugsweise ein maschinelles Geschirrspülmittel, zur Verfügung zu stellen, das bei solchen Anschmutzungen eine gesteigerte Reinigungsleistung aufweist, ohne dass die Reinigungsleistung an anderen Anschmutzungen verringert wird.

Überraschenderweise wurde nun festgestellt, dass die Verwendung einer Kombination verschiedener Proteasen die Reinigungsleistung von entsprechenden Geschirrspülmitteln, vorzugsweise eines maschinellen Geschirrspülmittels an protease-sensitiven Anschmutzungen, insbesondere angebrannten Lebensmittelanschmutzungen, insbesondere an angebrannten zuckerhaltigen Lebensmittelanschmutzungen, erheblich verbessert.

In einem ersten Aspekt richtet sich die vorliegende Erfindung daher auf ein Reinigungsmittel für harte Oberflächen, das ein Geschirrspülmittel, vorzugsweise ein maschinelles Geschirrspülmittel, ist, das eine erste und eine zweite Protease umfasst, wobei
a) die erste Protease eine Aminosäuresequenz gemäß SEQ ID NO:3 aufweist, und
b) die zweite Protease ausgewählt ist aus der Gruppe gebildet aus
b1) Protease, die eine Aminosäuresequenz umfasst, die zu der in SEQ ID NO. 2 angegebenen Aminosäuresequenz über deren Gesamtlänge zu mindestens 95% identisch ist und in der Zählung gemäß SEQ ID NO. 2 an Position 99 die Aminosäure Glutaminsäure (E) aufweist;
b2) Protease, die eine Aminosäuresequenz umfasst, die zu der in SEQ ID NO. 2 angegebenen Aminosäuresequenz über deren Gesamtlänge zu mindestens 95% identisch ist und in der Zählung gemäß SEQ ID NO. 2 die Aminosäuresubstitution R99E in Kombination mit mindestens zwei weiteren Aminosäuresubstitutionen aufweist, die ausgewählt sind aus der Gruppe bestehend aus S3T, V4I und V199I; und
wobei
die Protease gemäß b1) eine Aminosäuresequenz aufweist, die in der Zählung gemäß SEQ ID NO. 2 in den Positionen 1-98 und 100-269 SEQ ID NO. 2 entspricht und an Position 99 die Aminosäure Glutaminsäure (E) aufweist; und
die Protease b2) eine Aminosäuresequenz gemäß SEQ ID NO. 2 mit den Aminosäuresubstitutionen R99E, S3T, V4I und V199I in der Zählung gemäß SEQ ID NO. 2 aufweist.

Eine solche Kombination mehrerer Proteasen bewirkt bei Anwendung des Mittels eine signifikant gesteigerte Reinigungsleistung an hartnäckigen Anschmutzungen.

Ein weiterer Gegenstand der vorliegenden Erfindung ist die Verwendung eines solchen Geschirrspülmittels, vorzugsweise eines maschinellen Geschirrspülmittels in einem Reinigungsverfahren, bevorzugt einem Geschirrspülverfahren, insbesondere in einem maschinellen Geschirrspülverfahren, vorzugsweise die Verwendung zur Verbesserung der Reinigungsleistung, insbesondere der Reinigungsleistung an protease-sensitiven Anschmutzungen, an harten Oberflächen, insbesondere Geschirr bei dessen Reinigung, bevorzugt in einer automatischen Geschirrspülmaschine, insbesondere angebrannten, hartnäckigen Anschmutzungen, u.a. auch bei Temperaturen, die niedriger sind als die üblicherweise verwendeten Temperaturen.

Ein weiterer Gegenstand der vorliegenden Erfindung ist ein Geschirrspülverfahren, vorzugsweise ein maschinelles Geschirrspülverfahren, bei dem ein solches Geschirrspülmittel, vorzugsweise ein maschinelles Geschirrspülmittel, insbesondere zu dem Zweck, die Reinigungsleistung an angebrannten, protease-sensitiven Anschmutzungen zu verbessern, zum Einsatz kommt. In verschiedenen Ausführungsformen der Erfindung werden bei dem Geschirrspülverfahren Temperaturen eingesetzt, die niedriger sind als die üblicherweise verwendeten Temperaturen.

"Niedrige Temperaturen" oder "Temperaturen, die niedriger sind als die üblicherweise verwendeten Temperaturen", wie hierin im Zusammenhang mit Geschirrspülverfahren verwendet, bezieht sich vorzugsweise auf Temperaturen unter 60°C, insbesondere unter 55°C, noch bevorzugter 50°C oder niedriger, besonders bevorzugt 45°C oder niedriger und am bevorzugtesten 40°C oder niedriger. Diese Temperaturangaben beziehen sich auf die in den Spülschritten eingesetzten Temperaturen.

Diese und weitere Aspekte, Merkmale und Vorteile der Erfindung werden für den Fachmann aus dem Studium der folgenden detaillierten Beschreibung und Ansprüche ersichtlich. Dabei kann jedes Merkmal aus einem Aspekt der Erfindung in jedem anderen Aspekt der Erfindung eingesetzt werden. Ferner ist es selbstverständlich, dass die hierin enthaltenen Beispiele die Erfindung beschreiben und veranschaulichen sollen, diese aber nicht einschränken und insbesondere die Erfindung nicht auf diese Beispiele beschränkt ist. Alle Prozentangaben sind, sofern nicht anders angegeben, Gewichts-%. Numerische Bereiche, die in dem Format "von x bis y" angegeben sind, schließen die genannten Werte ein. Wenn mehrere bevorzugte numerische Bereiche in diesem Format angegeben sind, ist es selbstverständlich, dass alle Bereiche, die durch die Kombination der verschiedenen Endpunkte entstehen, ebenfalls erfasst werden.

Die eingesetzten Proteasen sind alkalische Serin-Proteasen. Sie wirken als unspezifische Endopeptidasen, das heißt, sie hydrolysieren beliebige Säureamidbindungen, die im Inneren von Peptiden oder Proteinen liegen und bewirken dadurch den Abbau proteinhaltiger Anschmutzungen auf dem Reinigungsgut. Ihr pH-Optimum liegt meist im deutlich alkalischen Bereich.

Die Sequenzen der reifen (maturen) Protease Subtilisin 309 aus Bacillus lentus, welche unter dem Handelsnamen Savinase^{®} von der Firma Novozymes A/S, Bagsvaerd, Dänemark vertrieben wird bzw. der reifen (maturen) alkalischen Protease aus Bacillus lentus DSM 5483 (wildtyp) sind in SEQ ID NO. 1 bzw. SEQ ID NO. 2 angegeben.

"Verschieden", wie hierin mit Bezug auf die Proteasen verwendet, bezieht sich auf Proteasen, die sich in ihrer Aminosäuresequenz unterscheiden. In verschiedenen Ausführungsformen stammen voneinander verschiedene Proteasen aus unterschiedlichen Arten von Organismen oder unterscheiden sich voneinander durch, beispielsweise künstlich erzeugte, Mutationen.

"Variante", wie hierin in Zusammenhang mit Proteasen verwendet, bezieht sich auf natürliche oder artifiziell erzeugte Variationen einer nativen Protease, die eine gegenüber der Referenzform abgewandelte Aminosäuresequenz aufweisen. Eine solche Variante kann einzelne oder mehrere Punktmutationen, d.h. Substitutionen einer natürlicherweise an der entsprechenden Position vorkommenden Aminosäure durch eine andere, Insertionen (Einfügen von einer oder mehreren Aminosäuren) und/oder Deletionen (Entfernen von einer oder mehreren Aminosäuren) aufweisen, insbesondere eine oder mehrere Punktmutationen. Derartige Varianten haben vorzugsweise mindestens 50, vorzugsweise 60 oder mehr, noch bevorzugter 70, 80, 90, 100 % oder mehr der Enzymaktivität der Referenzform. In verschiedenen Ausführungsformen hat eine derartige Variante eine Aminosäuresequenz, die zu der als Referenz dienenden Sequenz über deren Gesamtlänge zu mindestens 70, vorzugsweise 75, 80, 85, 90, 95, 96, 97, 98, oder 99 % identisch ist. Die Varianten haben vorzugsweise dieselbe Länge wie die Referenzsequenz. Varianten können sich gegenüber der Referenzform durch verbesserte Eigenschaften auszeichnen, wie beispielsweise höhere Enzymaktivität, höhere Stabilität, geänderte Substratspezifität, etc.. Eingesetzt werden nur solche Varianten, die Proteaseaktivität aufweisen.

Die Bestimmung der Identität von Nukleinsäure- oder Aminosäuresequenzen erfolgt durch einen Sequenzvergleich. Dieser Sequenzvergleich basiert auf dem im Stand der Technik etablierten und üblicherweise genutzten BLAST-Algorithmus (vgl. beispielsweise Altschul, S.F., Gish, W., Miller, W., Myers, E.W. & Lipman, D.J. (1990) "Basic local alignment search tool." J. Mol. Biol. 215:403-410, und Altschul, Stephan F., Thomas L. Madden, Alejandro A. Schaffer, Jinghui Zhang, Hheng Zhang, Webb Miller, and David J. Lipman (1997): "Gapped BLAST and PSI-BLAST: a new generation of protein database search programs"; Nucleic Acids Res., 25, S.3389-3402) und geschieht prinzipiell dadurch, dass ähnliche Abfolgen von Nukleotiden oder Aminosäuren in den Nukleinsäure- oder Aminosäuresequenzen einander zugeordnet werden. Eine tabellarische Zuordnung der betreffenden Positionen wird als Alignment bezeichnet. Ein weiterer im Stand der Technik verfügbarer Algorithmus ist der FASTA-Algorithmus.

Solch ein Vergleich erlaubt auch eine Aussage über die Ähnlichkeit der verglichenen Sequenzen zueinander. Sie wird üblicherweise in Prozent Identität, das heißt dem Anteil der identischen Nukleotide oder Aminosäurereste an denselben oder in einem Alignment einander entsprechenden Positionen angegeben. Der weiter gefasste Begriff der Homologie bezieht bei Aminosäuresequenzen konservierte Aminosäure-Austausche in die Betrachtung mit ein, also Aminosäuren mit ähnlicher chemischer Aktivität, da diese innerhalb des Proteins meist ähnliche chemische Aktivitäten ausüben. Daher kann die Ähnlichkeit der verglichenen Sequenzen auch Prozent Homologie oder Prozent Ähnlichkeit angegeben sein. Identitäts- und/oder Homologieangaben können über ganze Polypeptide oder Gene oder nur über einzelne Bereiche getroffen werden. Homologe oder identische Bereiche von verschiedenen Nukleinsäure- oder Aminosäuresequenzen sind daher durch Übereinstimmungen in den Sequenzen definiert. Solche Bereiche weisen oftmals identische Funktionen auf. Sie können klein sein und nur wenige Nukleotide oder Aminosäuren umfassen. Oftmals üben solche kleinen Bereiche für die Gesamtaktivität des Proteins essentielle Funktionen aus. Es kann daher sinnvoll sein, Sequenzübereinstimmungen nur auf einzelne, gegebenenfalls kleine Bereiche zu beziehen. Soweit nicht anders angegeben beziehen sich Identitäts- oder Homologieangaben in der vorliegenden Anmeldung aber auf die Gesamtlänge der jeweils angegebenen Nukleinsäure- oder Aminosäuresäuresequenz.

Als erste Protease im Sinne der vorliegenden Erfindung wird eine Protease eingesetzt, die eine Aminosäuresequenz gemäß SEQ ID NO. 3 aufweist.

Die zweite Protease ist von der ersten Protease verschieden, d.h. eine Protease, die sowohl unter die Definition für die erste Protease als auch für die der zweiten Protease fällt, kann nicht als erste und als zweite Protease gleichzeitig gezählt werden.

Die zweite Protease b) ist ausgewählt ist aus der Gruppe
b1) Protease, die eine Aminosäuresequenz umfasst, die zu der in SEQ ID NO. 2 angegebenen Aminosäuresequenz über deren Gesamtlänge zu mindestens 95% identisch ist und in der Zählung gemäß SEQ ID NO. 2 an Position 99 die Aminosäure Glutaminsäure (E) aufweist;
b2) Protease, die eine Aminosäuresequenz umfasst, die zu der in SEQ ID NO. 2 angegebenen Aminosäuresequenz über deren Gesamtlänge zu mindestens 95% identisch ist und in der Zählung gemäß SEQ ID NO. 2 die Aminosäuresubstitution R99E in Kombination mit mindestens zwei weiteren Aminosäuresubstitutionen aufweist, die ausgewählt sind aus der Gruppe bestehend aus S3T, V4I und V199I; wobei
die Protease gemäß b1) eine Aminosäuresequenz aufweist, die in der Zählung gemäß SEQ ID NO. 2 in den Positionen 1-98 und 100-269 SEQ ID NO. 2 entspricht und an Position 99 die Aminosäure Glutaminsäure (E) aufweist; und
die Protease b2) eine Aminosäuresequenz gemäß SEQ ID NO. 2 mit den Aminosäuresubstitutionen R99E, S3T, V4I und V199I in der Zählung gemäß SEQ ID NO. 2 aufweist.

Ein zweiter wesentlicher Bestandteil erfindungsgemäßer Geschirrspülmittel ist die Protease b), ausgewählt aus den Proteasen b1) und b2).

Erfindungsgemäße Geschirrspülmittel sind dadurch gekennzeichnet, dass die Protease b1) eine Aminosäuresequenz aufweist, die in der Zählung gemäß SEQ ID NO. 2 in den Positionen 1-98 und 100-269 SEQ ID NO. 2 entspricht und an Position 99 die Aminosäure Glutaminsäure (E) aufweist. Eine derartige Protease ist in SEQ ID NO. 4 angegeben.

Erfindungsgemäße Geschirrspülmittel sind dadurch gekennzeichnet, dass die Protease b2) eine Aminosäuresequenz gemäß SEQ ID NO. 2 mit den Aminosäuresubstitutionen R99E, S3T, V4I und V199I in der Zählung gemäß SEQ ID NO. 2 aufweist. Eine derartige Protease ist in SEQ ID NO. 5 angegeben.

Erfindungsgemäße Kombinationen von Proteasen sind insbesondere die Kombination der Protease mit der in SEQ ID NO:3 angegebenen Aminosäuresequenz mit einer Protease, die eine der in SEQ ID NO:4 oder SEQ ID NO:5 angegebenen Aminosäuresequenzen aufweist.

In verschiedenen Ausführungsformen werden diese Kombinationen von Proteasen im Massenverhältnis bezogen auf Aktivprotein von 10:1 bis 1:10, vorzugsweise 5:1 bis 1:5, eingesetzt. Besonders bevorzugt wird die Protease mit der in SEQ ID NO:3 angegebenen Aminosäuresequenz mit der Protease mit der in SEQ ID NO:4 angegebenen Aminosäuresequenz in einem Masseverhältnis von 10:1 bis 1:10, vorzugsweise 5:1 bis 1:5, insbesondere von 2:1 bis 1:2, besonders bevorzugt zu gleichen Teilen eingesetzt. Weiterhin besonders bevorzugt wird die Protease mit der in SEQ ID NO:3 angegebenen Aminosäuresequenz mit der Protease mit der in SEQ ID NO:5 angegebenen Aminosäuresequenz in einem Masseverhältnis von 10:1 bis 1:10, vorzugsweise 5:1 bis 1:5, insbesondere von 2:1 bis 1:2, besonders bevorzugt zu gleichen Teilen eingesetzt.

Überraschenderweise zeigen die hierin beschriebenen Kombinationen von verschiedenen Proteasen die Eigenschaft, die Leistung des Geschirrspülmittels zu verbessern, indem sie zu einer verbesserten Reinigungsleistung an hartnäckigen, angebrannten Anschmutzungen führen. Die Steigerung der Reinigungsleistung ist auch bei niedrigen Temperaturen, d.h. Temperaturen, die niedriger sind als die üblicherweise in Geschirrspülverfahren verwendeten, wie oben definiert, zu beobachten. Das ermöglicht es, das Reinigungsverfahren, bevorzugt das automatische Geschirrspülverfahren bei niedrigeren Temperaturen durchzuführen und trotzdem eine gute Reinigungsleistung beizubehalten.

Dabei ist in der Regel unter der Verbesserung der Reinigungsleistung zu verstehen, dass bei Verwendung der hierin beschriebenen Geschirrspülmittel die Entfernung von Anschmutzungen, insbesondere angebrannten Anschmutzungen, auf harten Oberflächen, insbesondere Geschirr, bei dessen Reinigung bevorzugt in einer automatischen Geschirrspülmaschine im Vergleich zu der Verwendung von Geschirrspülmitteln, die die hierin beschriebenen Enzymkombinationen nicht enthalten, merklich verbessert ist.

Die einzusetzenden Enzyme können ferner zusammen mit Begleitstoffen, etwa aus der Fermentation, oder mit Stabilisatoren konfektioniert sein.

Die Geschirrspülmittel, enthalten jede Protease vorzugsweise in einer Menge von 1 x 10₋₈ bis 5 Gew.-% bezogen auf das jeweilige aktive Protein. Bevorzugt sind die Enzyme jeweils von 0,001 bis 2 Gew.-%, weiter bevorzugt von 0,005 bis 1,5 Gew.-%, noch weiter bevorzugt von 0,01 bis 1 Gew.-% und besonders bevorzugt von 0,01 bis 0,5 Gew.-% in diesen Mitteln enthalten.

In besonders bevorzugten Ausführungsformen wird in den hierin beschriebenen Mitteln die erste Protease in einer Gesamtmenge vom 0,01 bis 1 Gew.-%, vorzugsweise 0,025 bis 0,5 Gew.-% bezogen auf aktives Protein eingesetzt. In gleicher Weise wird vorzugsweise die zweite Protease in einer Gesamtmenge von 0,005 bis 0,75 Gew.-%, vorzugsweise 0,01 bis 0,5 Gew.-% bezogen auf aktives Protein eingesetzt. Als erste Protease wird bevorzugt 0,025 bis 0,5 Gew.-% der Protease mit der Sequenz von SEQ ID NO:3 und als zweite Protease 0,01 bis 0,5 Gew.-% der Protease mit der Sequenz von SEQ ID NO:4 eingesetzt. In einer weiteren bevorzugten Ausführungsform wird als erste Protease bevorzugt 0,025 bis 0,5 Gew.-% der Protease mit der Sequenz von SEQ ID NO:3 und als zweite Protease 0,01 bis 0,5 Gew.-% der Protease mit der Sequenz von SEQ ID NO:5 eingesetzt.

In flüssigen Formulierungen werden die Enzyme bevorzugt als Enzymflüssigformulierung(en) eingesetzt.

Die Proteinkonzentration kann mit Hilfe bekannter Methoden, zum Beispiel dem BCA-Verfahren (Bicinchoninsäure; 2,2'-Bichinolyl-4,4'-dicarbonsäure) oder dem Biuret-Verfahren bestimmt werden. Die Bestimmung der Aktivproteinkonzentration erfolgt diesbezüglich über eine Titration der aktiven Zentren unter Verwendung eines geeigneten irreversiblen Inhibitors (für Proteasen beispielsweise Phenylmethylsulfonylfluorid (PMSF)) und Bestimmung der Restaktivität (vgl. M. Bender et al., J. Am. Chem. Soc. 88, 24 (1966), S. 5890-5913).

Die Proteasen können besonders während der Lagerung gegen Schädigungen wie beispielsweise Inaktivierung, Denaturierung oder Zerfall etwa durch physikalische Einflüsse, Oxidation oder proteolytische Spaltung geschützt werden. Bei mikrobieller Gewinnung ist eine Inhibierung der Proteolyse besonders bevorzugt. Die beschriebenen Mittel können zu diesem Zweck Stabilisatoren enthalten.

Reinigungsaktive Proteasen werden in der Regel nicht in Form des reinen Proteins sondern vielmehr in Form stabilisierter, lager- und transportfähiger Zubereitungen bereitgestellt. Zu diesen vorkonfektionierten Zubereitungen zählen beispielsweise die durch Granulation, Extrusion oder Lyophilisierung erhaltenen festen Präparationen oder, insbesondere bei flüssigen oder gelförmigen Mitteln, Lösungen der Enzyme, vorteilhafterweise möglichst konzentriert, wasserarm und/oder mit Stabilisatoren oder weiteren Hilfsmitteln versetzt.

Alternativ können die Enzyme sowohl für die feste als auch für die flüssige Darreichungsform verkapselt werden, beispielsweise durch Sprühtrocknung oder Extrusion der Enzymlösung zusammen mit einem vorzugsweise natürlichen Polymer oder in Form von Kapseln, beispielsweise solchen, bei denen die Enzyme wie in einem erstarrten Gel eingeschlossen sind oder in solchen vom Kern-Schale-Typ, bei dem ein enzymhaltiger Kern mit einer Wasser-, Luft- und/oder Chemikalien-undurchlässigen Schutzschicht überzogen ist. In aufgelagerten Schichten können zusätzlich weitere Wirkstoffe, beispielsweise Stabilisatoren, Emulgatoren, Pigmente, Bleich- oder Farbstoffe aufgebracht werden. Derartige Kapseln werden nach an sich bekannten Methoden, beispielsweise durch Schüttel- oder Rollgranulation oder in Fluidbed-Prozessen aufgebracht. Vorteilhafterweise sind derartige Granulate, beispielsweise durch Aufbringen polymerer Filmbildner, staubarm und aufgrund der Beschichtung lagerstabil.

Weiterhin ist es möglich, zwei oder mehrere Enzyme zusammen zu konfektionieren, so dass ein einzelnes Granulat mehrere Enzymaktivitäten aufweist.

Wie aus der vorherigen Ausführungen ersichtlich, bildet das Enzym-Protein nur einen Bruchteil des Gesamtgewichts üblicher Enzym-Zubereitungen. Bevorzugt eingesetzte Protease-Zubereitungen enthalten zwischen 0,1 und 40 Gew.-%, bevorzugt zwischen 0,2 und 30 Gew.-%, besonders bevorzugt zwischen 0,4 und 20 Gew.-% und insbesondere zwischen 0,8 und 15 Gew.-% des Enzymproteins. Die beschriebenen Mittel enthalten derartige Enzym-Zubereitungen daher vorzugsweise in einer Menge von jeweils 0,1 bis 10 Gew.-%, vorzugsweise 0,2 bis 5 Gew.-% bezogen auf das Gesamtmittel.

Die hierin beschriebenen Geschirrspülmittel, insbesondere die bevorzugten maschinellen Geschirrspülmittel können fester oder flüssiger Natur sein und insbesondere als pulverförmige Feststoffe, in nachverdichteter Teilchenform, als homogene Lösungen oder Suspensionen vorliegen. In einer weiteren bevorzugten Ausführungsform der Erfindung liegt das maschinelle Geschirrspülmittel in einer vorportionierten Form vor. In einer weiteren bevorzugten Ausführungsform der Erfindung weist das maschinelle Geschirrspülmittel mehrere räumlich voneinander getrennte Zusammensetzungen auf, wodurch es möglich ist, nicht kompatible Inhaltsstoffe voneinander zu trennen, oder Zusammensetzungen in Kombination anzubieten, welche zu unterschiedlichen Zeitpunkten in der Geschirrspülmaschine zum Einsatz kommen. Dies ist besonders vorteilhaft, wenn die maschinellen Geschirrspülmittel in vorportionierter Form vorliegen. Dabei kann mindestens eine der Zusammensetzungen fest und/oder mindestens eine der Zusammensetzungen flüssig vorliegen, wobei die Proteasen in mindestens einer der Zusammensetzungen enthalten sind, aber auch in mehreren Zusammensetzungen vorliegen können.

Vorzugsweise enthalten die hierin beschriebenen Geschirrpülmittel mindestens einen weiteren Bestandteil, insbesondere mindestens zwei weitere Bestandteile, ausgewählt aus der Gruppe bestehend aus Gerüststoffen, Tensiden, Polymeren, Bleichmitteln, Bleichkatalysatoren, Bleichaktivatoren, Nicht-Protease-Enzymen, Korrosionsinhibitoren und Glaskorrosionsinhibitoren, Desintegrationshilfsmitteln, Duftstoffen und Parfümträgern.

Nachfolgend werden mögliche Inhaltsstoffe beschrieben, welche vorteilhafterweise in den hierin beschriebenen Geschirrspülmittel, bevorzugt maschinellen Geschirrspülmitteln eingesetzt werden können.

Vorteilhafterweise können Gerüststoffe eingesetzt werden. Zu den Gerüststoffen zählen insbesondere die Zeolithe, Silikate, Carbonate, organische Cobuilder und -wo keine ökologischen Vorurteile gegen ihren Einsatz bestehen - auch die Phosphate.

In den hierin beschriebenen Mitteln können kristalline schichtförmige Silikate eingesetzt werden. Solche Geschirrspülmittel, insbesondere maschinellen Geschirrspülmittel enthalten vorzugsweise einen Gewichtsanteil an kristallinem schichtförmigen Silikat von 0,1 bis 20 Gew.-%, bevorzugt von 0,2 bis 15 Gew.-% und insbesondere von 0,4 bis 10 Gew.-%, jeweils bezogen auf das Gesamtgewicht dieser Mittel.

Auch ein Einsatz der allgemein bekannten Phosphate als Buildersubstanzen ist möglich, sofern ein derartiger Einsatz nicht aus ökologischen Gründen vermieden werden sollte. Unter der Vielzahl der kommerziell erhältlichen Phosphate haben die Alkalimetallphosphate unter besonderer Bevorzugung von Pentanatrium- bzw. Pentakaliumtriphosphat (Natrium- bzw. Kaliumtripolyphosphat) in der Wasch- oder Reinigungsmittel-Industrie die größte Bedeutung.

Alkalimetallphosphate ist dabei die summarische Bezeichnung für die Alkalimetall- (insbesondere Natriumund Kalium-) Salze der verschiedenen Phosphorsäuren, bei denen man Metaphosphorsäuren (HPO₃)ₙ und Orthophosphorsäure H₃PO₄ neben höhermolekularen Vertretern unterscheiden kann. Die Phosphate vereinen dabei mehrere Vorteile in sich: Sie wirken als Alkaliträger, verhindern Kalkbeläge auf Maschinenteilen bzw. Kalkinkrustationen in Geweben und tragen überdies zur Reinigungsleistung bei.

Technisch besonders wichtige Phosphate sind das Pentanatriumtriphosphat, Na₅P₃O₁₀ (Natriumtripolyphosphat) sowie das entsprechende Kaliumsalz Pentakaliumtriphosphat, K₅P₃O₁₀ (Kaliumtripolyphosphat). Bevorzugt einsetzbar sind die Natriumkaliumtripolyphosphate.

Werden im Rahmen der vorliegenden Anmeldung Phosphate als wasch- oder reinigungsaktive Substanzen in den Geschirrspülmitteln, insbesondere im maschinellen Geschirrspülmittel eingesetzt, so enthalten bevorzugte Mittel diese(s) Phosphat(e), vorzugsweise Alkalimetallphosphat(e), besonders bevorzugt Pentanatrium- bzw. Pentakaliumtriphosphat (Natrium- bzw. Kaliumtripolyphosphat), in Mengen von 5 bis 80 Gew.-%, vorzugsweise von 15 bis 75 Gew.-% und insbesondere von 20 bis 70 Gew.-%, jeweils bezogen auf das Gewicht des Geschirrspülmittels, insbesondere maschinellen Geschirrspülmittels.

Weitere Gerüststoffe sind die Alkaliträger. Als Alkaliträger gelten beispielsweise Alkalimetallhydroxide, Alkalimetallcarbonate, Alkalimetallhydrogencarbonate, Alkalimetallsesquicarbonate, die genannten Alkalisilikate, Alkalimetasilikate, und Mischungen der vorgenannten Stoffe, wobei im Sinne dieser Erfindung bevorzugt die Alkalicarbonate, insbesondere Natriumcarbonat, Natriumhydrogencarbonat oder Natriumsesquicarbonat eingesetzt werden können. Besonders bevorzugt ist ein Buildersystem enthaltend eine Mischung aus Tripolyphosphat und Natriumcarbonat. Ebenfalls besonders bevorzugt ist ein Buildersystem enthaltend eine Mischung aus Tripolyphosphat und Natriumcarbonat und Natriumdisilikat. Aufgrund ihrer im Vergleich mit anderen Buildersubstanzen geringen chemischen Kompatibilität mit den übrigen Inhaltsstoffen von Geschirrspülmitteln, bevorzugt maschinellen Geschirrspülmitteln, werden die optionalen Alkalimetallhydroxide bevorzugt nur in geringen Mengen oder überhaupt nicht eingesetzt.

Besonders bevorzugt ist der Einsatz von Carbonat(en) und/oder Hydrogencarbonat(en), vorzugsweise Alkalicarbonat(en), besonders bevorzugt Natriumcarbonat, in Mengen von 2 bis 50 Gew.-%, vorzugsweise von 5 bis 40 Gew.-% und insbesondere von 7,5 bis 30 Gew.-%, jeweils bezogen auf das Gewicht des Geschirrspülmittels, bevorzugt maschinellen Geschirrspülmittels. Besonders bevorzugt werden Geschirrspülmittel, welche bezogen auf das Gewicht des maschinellen Geschirrspülmittels weniger als 20 Gew.-%, vorzugsweise weniger als 17 Gew.-%, bevorzugt weniger als 13 Gew.-% und insbesondere weniger als 9 Gew.-% Carbonat(e) und/oder Hydrogencarbonat(e), vorzugsweiseAlkalicarbonat(e), besonders bevorzugt Natriumcarbonat enthalten.

Als organische Cobuilder sind insbesondere Polycarboxylate / Polycarbonsäuren, polymere Polycarboxylate, Asparaginsäure, Polyacetale, Dextrine, weitere organische Cobuildersowie Phosphonate zu nennen. Diese Stoffklassen werden nachfolgend beschrieben.

Brauchbare organische Gerüstsubstanzen sind beispielsweise die in Form der freien Säure und/oder ihrer Natriumsalze einsetzbaren Polycarbonsäuren, wobei unter Polycarbonsäuren solche Carbonsäuren verstanden werden, die mehr als eine Säurefunktion tragen. Beispielsweise sind dies Citronensäure, Adipinsäure, Bernsteinsäure, Glutarsäure, Äpfelsäure, Weinsäure, Maleinsäure, Fumarsäure, Zuckersäuren, Nitrilotriessigsäure (NTA), sofern ein derartiger Einsatz aus ökologischen Gründen nicht zu beanstanden ist, sowie Mischungen aus diesen. Die freien Säuren besitzen neben ihrer Builderwirkung typischerweise auch die Eigenschaft einer Säuerungskomponente und dienen somit auch zur Einstellung eines niedrigeren und milderen pH-Wertes Mittels. Insbesondere sind hierbei Citronensäure, Bernsteinsäure, Glutarsäure, Adipinsäure, Gluconsäure und beliebige Mischungen aus diesen zu nennen.

Als besonders vorteilhaft für die Reinigungs- und Klarspülleistung hierin beschriebener Mittel hat sich der Einsatz von Citronensäure und/oder Citraten in diesen Mitteln erwiesen. Bevorzugt werden daher Geschirrspülmittel, insbesondere bevorzugt maschinelle Geschirrspülmittel, dadurch gekennzeichnet, dass das Mittel Citronensäure oder ein Salz der Citronensäure enthält und das der Gewichtsanteil der Citronensäure oder des Salzes der Citronensäure vorzugsweise mehr als 10 Gew.-%, bevorzugt mehr als 15 Gew.-% und insbesondere zwischen 20 und 40 Gew.-% beträgt. Salze dar. Besonders bevorzugte Vertreter dieser Klasse sind Methylglycindiessigsäure (MGDA) oder ihre Salze sowie Glutamindiessigsäure (GLDA) oder ihre Salze oder Ethylendiamindiessigsäure oder ihre Salze (EDDS). Der Gehalt an diesen Aminocarbonsäuren bzw. ihren Salzen kann beispielsweise zwischen 0,1 und 15 Gew.-% bevorzugt zwischen 0,5 und 10 Gew.-% und insbesondere zwischen 0,5 und 6 Gew.-% ausmachen. Aminocarbonsäuren und ihre Salze können zusammen mit den vorgenannten Gerüststoffen, insbesondere auch mit den phosphatfreien Gerüststoffen eingesetzt werden.

Eine weitere bedeutende Klasse der phosphatfreien Gerüststoffe stellen Aminocarbonsäuren und/oder ihre Salze dar. Besonders bevorzugte Vertreter dieser Klasse sind Methylglycindiessigsäure (MGDA) oder ihre Salze sowie Glutamindiessigsäure (GLDA) oder ihre Salze oder Ethylendiamindiessigsäure oder ihre Salze (EDDS). Der Gehalt an diesen Aminocarbonsäuren bzw. ihren Salzen kann beispielsweise zwischen 0,1 und 15 Gew.-% bevorzugt zwischen 0,5 und 10 Gew.-% und insbesondere zwischen 0,5 und 6 Gew.-% ausmachen. Aminocarbonsäuren und ihre Salze können zusammen mit den vorgenannten Gerüststoffen, insbesondere auch mit den phosphatfreien Gerüststoffen eingesetzt werden.

Als Gerüststoffe sind weiter polymere Polycarboxylate geeignet, dies sind beispielsweise die Alkalimetallsalze der Polyacrylsäure oder der Polymethacrylsäure, beispielsweise solche mit einer relativen Molekülmasse von 500 bis 70000 g/mol. Geeignete Polymere sind insbesondere Polyacrylate, die bevorzugt eine Molekülmasse von 2000 bis 20000 g/mol aufweisen. Aufgrund ihrer überlegenen Löslichkeit können aus dieser Gruppe wiederum die kurzkettigen Polyacrylate, die Molmassen von 2000 bis 10000 g/mol, und besonders bevorzugt von 3000 bis 5000 g/mol, aufweisen, bevorzugt sein.

Geeignet sind weiterhin copolymere Polycarboxylate, insbesondere solche der Acrylsäure mit Methacrylsäure und der Acrylsäure oder Methacrylsäure mit Maleinsäure.

Die (co-)polymeren Polycarboxylate können entweder als Pulver oder als wässrige Lösung eingesetzt werden. Der Gehalt der Geschirrspülmittel, insbesondere maschinellen Geschirrspülmittel an (co-)polymeren Polycarboxylaten beträgt vorzugsweise 0,5 bis 20 Gew.-% und insbesondere 3 bis 10 Gew.-%.

Zur Verbesserung der Wasserlöslichkeit können die Polymere auch Allylsulfonsäuren, wie beispielsweise Allyloxybenzolsulfonsäure und Methallylsulfonsäure, als Monomer enthalten. Weitere bevorzugte Copolymere sind solche, die als Monomere Acrolein und Acrylsäure/Acrylsäuresalze bzw. Acrolein und Vinylacetat aufweisen.

Darüber hinaus können alle Verbindungen, die in der Lage sind, Komplexe mit Erdalkaliionen auszubilden, als Gerüststoffe eingesetzt werden.

Die hierin beschriebenen Mittel können Tenside enthalten, wobei zur Gruppe der Tenside die nichtionischen, die anionischen, die kationischen und die amphoteren Tenside gezählt werden.

Als nichtionische Tenside können alle dem Fachmann bekannten nichtionischen Tenside eingesetzt werden. Als nichtionische Tenside eignen sich beispielsweise Alkylglykoside der allgemeinen Formel RO(G)ₓ, in der R einem primären geradkettigen oder methylverzweigten, insbesondere in 2-Stellung methylverzweigten aliphatischen Rest mit 8 bis 22, vorzugsweise 12 bis 18 C-Atomen entspricht und G das Symbol ist, das für eine Glykoseeinheit mit 5 oder 6 C-Atomen, vorzugsweise für Glucose, steht. Der Oligomerisierungsgrad x, der die Verteilung von Monoglykosiden und Oligoglykosiden angibt, ist eine beliebige Zahl zwischen 1 und 10; vorzugsweise liegt x bei 1,2 bis 1,4.

Eine weitere Klasse bevorzugt einsetzbarer nichtionischer Tenside, die entweder als alleiniges nichtionisches Tensid oder in Kombination mit anderen nichtionischen Tensiden eingesetzt werden können, sind alkoxylierte, vorzugsweise ethoxylierte oder ethoxylierte und propoxylierte Fettsäurealkylester, vorzugsweise mit 1 bis 4 Kohlenstoffatomen in der Alkylkette.

Auch nichtionische Tenside vom Typ der Aminoxide, beispielsweise N-Kokosalkyl-N,N-dimethylaminoxid und N-Talgalkyl-N,N-dihydroxyethylaminoxid, und der Fettsäurealkanolamide können geeignet sein. Die Menge dieser nichtionischen Tenside beträgt vorzugsweise nicht mehr als die der ethoxylierten Fettalkohole, insbesondere nicht mehr als die Hälfte davon.

Weitere geeignete Tenside sind die als PHFA bekannten Polyhydroxyfettsäureamide.

Als bevorzugte Tenside können schwachschäumende nichtionische Tenside eingesetzt werden. Mit besonderem Vorzug enthalten die Geschirrspülmittel, insbesondere maschinellen Geschirrspülmittel nichtionische Tenside aus der Gruppe der alkoxylierten Alkohole. Als nichtionische Tenside werden vorzugsweise alkoxylierte, vorteilhafterweise ethoxylierte, insbesondere primäre Alkohole mit vorzugsweise 8 bis 18 C-Atomen und durchschnittlich 1 bis 12 Mol Ethylenoxid (EO) pro Mol Alkohol eingesetzt, in denen der Alkoholrest linear oder bevorzugt in 2-Stellung methylverzweigt sein kann bzw. lineare und methylverzweigte Reste im Gemisch enthalten kann, so wie sie üblicherweise in Oxoalkoholresten vorliegen. Insbesondere sind jedoch Alkoholethoxylate mit linearen Resten aus Alkoholen nativen Ursprungs mit 12 bis 18 C-Atomen, z.B. aus Kokos-, Palm-, Talgfett- oder Oleylalkohol, und durchschnittlich 2 bis 8 Mol EO pro Mol Alkohol bevorzugt. Zu den bevorzugten ethoxylierten Alkoholen gehören beispielsweise C₁₂₋₁₄-Alkohole mit 3 EO oder 4 EO, C₉₋₁₁-Alkohol mit 7 EO, C₁₃₋₁₅-Alkohole mit 3 EO, 5 EO, 7 EO oder 8 EO, C₁₂₋₁₈-Alkohole mit 3 EO, 5 EO oder 7 EO und Mischungen aus diesen, wie Mischungen aus C₁₂₋₁₄-Alkohol mit 3 EO und C₁₂₋₁₈-Alkohol mit 5 EO. Die angegebenen Ethoxylierungsgrade stellen statistische Mittelwerte dar, die für ein spezielles Produkt einer ganzen oder einer gebrochenen Zahl entsprechen können. Bevorzugte Alkoholethoxylate weisen eine eingeengte Homologenverteilung auf (narrow range ethoxylates, NRE). Zusätzlich zu diesen nichtionischen Tensiden können auch Fettalkohole mit mehr als 12 EO eingesetzt werden. Beispiele hierfür sind Talgfettalkohol mit 14 EO, 25 EO, 30 EO oder 40 EO.

Insbesondere bevorzugt sind nichtionische Tenside, die einen Schmelzpunkt oberhalb Raumtemperatur aufweisen. Nichtionische(s) Tensid(e) mit einem Schmelzpunkt oberhalb von 20°C, vorzugsweise oberhalb von 25°C, besonders bevorzugt zwischen 25 und 60°C und insbesondere zwischen 26,6 und 43,3°C, ist/sind besonders bevorzugt.

Bevorzugt einzusetzende Tenside stammen aus den Gruppen der alkoxylierten Niotenside, insbesondere der ethoxylierten primären Alkohole.

Aniontenside können ebenfalls als Bestandteil von Geschirrspülmitteln, insbesondere von maschinellen Geschirrspülmitteln eingesetzt werden. Zu ihnen zählen insbesondere Alkylbenzolsulfonate, (Fett-)Alkylsulfate, (Fett-)Alkylethersulfate sowie Alkansulfonate. Der Gehalt der Mittel an Aniontensiden beträgt üblicherweise 0 bis 10 Gew.-%.

An Stelle der genannten Tenside oder in Verbindung mit ihnen können auch kationische und/oder amphotere Tenside eingesetzt werden. In Geschirrspülmitteln, bevorzugt maschinellen Geschirrspülmitteln, beträgt der Gehalt an kationischen und/oder amphoteren Tensiden vorzugsweise weniger als 6 Gew.-%, bevorzugt weniger als 4 Gew.-%, ganz besonders bevorzugt weniger als 2 Gew.-% und insbesondere weniger als 1 Gew.-%. Mittel, welche keine kationischen oder amphoteren Tenside enthalten, werden besonders bevorzugt.

Zur Gruppe der Polymere zählen insbesondere die wasch- oder reinigungsaktiven Poylmere, beispielsweise die Klarspülpolymere und/oder als Enthärter wirksame Polymere. Generell sind in maschinellen Geschirrspülmitteln neben nichtionischen Polymeren auch kationische, anionische und amphotere Polymere einsetzbar.

"Kationische Polymere" im Sinne der vorliegenden Erfindung sind Polymere, welche eine positive Ladung im Polymermolekül tragen. Diese kann beispielsweise durch in der Polymerkette vorliegende (Alkyl-)Ammoniumgruppierungen oder andere positiv geladene Gruppen realisiert werden. Besonders bevorzugte kationische Polymere stammen aus den Gruppen der quaternierten Cellulose-Derivate, der Polysiloxane mit quaternären Gruppen, der kationischen Guar-Derivate, der polymeren Dimethyldiallylammoniumsalze und deren Copolymere mit Estern und Amiden von Acrylsäure und Methacrylsäure, der Copolymere des Vinylpyrrolidons mit quaternierten Derivaten des Dialkylaminoacrylats und -methacrylats, der Vinylpyrrolidon-Methoimidazoliniumchlorid-Copolymere, der quaternierter Polyvinylalkohole oder der unter den INCI-Bezeichnungen Polyquaternium 2, Polyquaternium 17, Polyquaternium 18 und Polyquaternium 27 angegeben Polymere.

"Amphotere Polymere" im Sinne der vorliegenden Erfindung weisen neben einer positiv geladenen Gruppe in der Polymerkette weiterhin auch negativ geladenen Gruppen bzw. Monomereinheiten auf. Bei diesen Gruppen kann es sich z.B. um Carbonsäuren, Sulfonsäuren oder Phosphonsäuren handeln.

Bevorzugte einsetzbare amphotere Polymere stammen aus der Gruppe der Alkylacrylamid/Acrylsäure-Copolymere, der Alkylacrylamid/Methacrylsäure-Copolymere, der Alkylacrylamid/Methylmethacrylsäure-Copolymere, der Alkylacrylamid/Acrylsäure/Alkylaminoalkyl(meth)acrylsäure-Copolymere, der Alkylacrylamid/Methacrylsäure/Alkylaminoalkyl(meth)-acrylsäure-Copolymere, der Alkylacrylamid/Methylmethacrylsäure/Alkylaminoalkyl(meth)acrylsäure-Copolymere, der Alkylacrylamid/Alkymethacrylat/Alkylaminoethylmethacrylat/Alkylmethacrylat-Copolymere sowie der Copolymere aus ungesättigten Carbonsäuren, kationisch derivatisierten ungesättigten Carbonsäuren und gegebenenfalls weiteren ionischen oder nichtionogenen Monomeren.

Bevorzugt einsetzbare zwitterionische Polymere stammen aus der Gruppe der Acrylamidoalkyltrialkylammoniumchlorid/Acrylsäure-Copolymere sowie deren Alkali- und Ammoniumsalze, der Acrylamidoalkyltrialkylammoniumchlorid/Methacrylsäure-Copolymere sowie deren Alkali- und Ammoniumsalze und der Methacroylethylbetain/Methacrylat-Copolymere.

Geschirrspülmittel, bevorzugt maschinelle Geschirrspülmittel enthalten die vorgenannten kationischen und/oder amphoteren Polymere vorzugsweise in Mengen zwischen 0,01 und 10 Gew.-%, jeweils bezogen auf das Gesamtgewicht des maschinellen Geschirrspülmittels. Bevorzugt werden im Rahmen der vorliegenden Anmeldung jedoch solche maschinelle Geschirrspülmittel, bei denen der Gewichtsanteil der kationischen und/oder amphoteren Polymere zwischen 0,01 und 8 Gew.-%, vorzugsweise zwischen 0,01 und 6 Gew.-%, bevorzugt zwischen 0,01 und 4 Gew.-%, besonders bevorzugt zwischen 0,01 und 2 Gew.-% und insbesondere zwischen 0,01 und 1 Gew.-%, jeweils bezogen auf das Gesamtgewicht des maschinellen Geschirrspülmittels, beträgt.

In den Geschirrspülmittel, insbesondere maschinellen Geschirrspülmitteln können ferner Bleichmittel eingesetzt werden. Unter den als Bleichmittel dienenden, in Wasser H₂O₂ liefernden Verbindungen haben das Natriumpercarbonat, das Natriumperborattetrahydrat und das Natriumperboratmonohydrat besondere Bedeutung. Weitere brauchbare Bleichmittel sind beispielsweise Peroxypyrophosphate, Citratperhydrate sowie H₂O₂ liefernde persaure Salze oder Persäuren, wie Perbenzoate, Peroxophthalate, Diperazelainsäure, Phthaloiminopersäure oder Diperdodecandisäure. Einsetzbar sind außerdem alle weiteren dem Fachmann aus dem Stand der Technik bekannten anorganischen oder organischen Peroxybleichmittel.

Als Bleichmittel können auch Chlor oder Brom freisetzende Substanzen eingesetzt werden. Unter den geeigneten Chlor oder Brom freisetzenden Materialien kommen beispielsweise heterozyklische N-Brom- und N-Chloramide, beispielsweise Trichlorisocyanursäure, Tribromisocyanursäure, Dibromisocyanursäure und/oder Dichlorisocyanursäure (DICA) und/oder deren Salze mit Kationen wie Kalium und Natrium in Betracht. Hydantoinverbindungen, wie 1,3-Dichlor-5,5-dimethylhydanthoin sind ebenfalls geeignet.

Es werden Geschirrspülmittel, insbesondere maschinelle Geschirrspülmittel bevorzugt, die 1 bis 35 Gew.-%, vorzugsweise 2,5 bis 30 Gew.-%, besonders bevorzugt 3,5 bis 20 Gew.-% und insbesondere 5 bis 15 Gew.-% Bleichmittel, vorzugsweise Natriumpercarbonat, enthalten.

Ferner können die Geschirrspülmittel, insbesondere maschinellen Geschirrspülmittel Bleichkatalysatoren enthalten. Die einsetzbaren Bleichkatalysatoren schließen ein, sind aber nicht beschränkt auf die Gruppe der bleichverstärkenden Übergangsmetallsalze und Übergangsmetallkomplexe, vorzugsweise der Mn-, Fe-, Co-, Ru - oder Mo-Komplexe, besonders bevorzugt aus der Gruppe der Mangan und/oder Cobaltsalze und/oder -komplexe, insbesondere der Cobalt(ammin)-Komplexe, der Cobalt(acetat)-Komplexe, der Cobalt(Carbonyl)-Komplexe, der Chloride des Cobalts oder Mangans, des Mangansulfats und der Komplexe des Mangans mit 1,4,7-trimethyl-1,4,7-triazacyclononan (Mn₃-TACN) oder 1,2,4,7-tetramethyl-1,4,7-tri-azacyclononan (Mn₄-TACN).

Es werden Geschirrspülmittel, insbesondere maschinelle Geschirrspülmittel bevorzugt, die 0,001 bis 1 Gew.-%, vorzugsweise 0,01 bis 0,1 Gew.-% Bleichkatalysator, vorzugsweise einen Mn-Komplex, insbesondere einen Komplex des Mangans mit 1,4,7-trimethyl-1,4,7-triazacyclononan (Mn₃-TACN) oder 1,2,4,7-tetramethyl-1,4,7-tri-azacyclononan (Mn₄-TACN), enthalten.

In verschiedenen Ausführungsformen der Erfindung enthalten die Geschirrspülmittel, insbesondere maschinellen Geschirrspülmittel zusätzlich mindestens einen Bleichaktivator. Als Bleichaktivatoren können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Von allen dem Fachmann aus dem Stand der Technik bekannten Bleichaktivatoren werden mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin (DADHT), acylierte Glykolurile, insbesondere Tetraacetylglykoluril (TAGU), N-Acylimide, insbesondere N-Nonanoylsuccinimid (NOSI), acylierte Phenolsulfonate, insbesondere n-Nonanoyl- oder Isononanoyloxybenzolsulfonat (n- bzw. iso-NOBS) besonders bevorzugt eingesetzt. Auch Kombinationen konventioneller Bleichaktivatoren können eingesetzt werden. Diese Bleichaktivatoren werden vorzugsweise in Mengen bis 10 Gew.-%, insbesondere 0,1 Gew.-% bis 8 Gew.-%, besonders 2 bis 8 Gew.-% und besonders bevorzugt 2 bis 6 Gew.-%, jeweils bezogen auf das Gesamtgewicht der bleichaktivatorhaltigen Mittel, eingesetzt.

Vorzugsweise enthalten die Geschirrspülmittel der vorliegenden Erfindung mindestens eine zusätzliche Enzymzubereitung oder Enzymzusammensetzung, die ein oder mehrere Nicht-Protease-Enzyme enthält. Solche Enzyme umfassen, ohne darauf beschränkt zu sein, Amylasen, Lipasen, Cellulasen, Hemicellulasen, Mannanasen, Pektin-spaltende Enzyme, Tannasen, Xylanasen, Xanthanasen, ^{®}-Glucosidasen, Carrageenasen, Perhydrolasen, Oxidasen, Oxidoreduktasen sowie deren Gemische. Bevorzugte Enzyme umfassen insbesondere Amylasen, insbesondere 〈-Amylasen, Cellulasen, Lipasen, Hemicellulasen, insbesondere Pectinasen, Mannanasen, ^{®}-Glucanasen, sowie deren Gemische. Besonders bevorzugt sind Amylasen und/oder Lipasen sowie deren Gemische. Diese Enzyme sind im Prinzip natürlichen Ursprungs; ausgehend von den natürlichen Molekülen stehen für den Einsatz in Geschirrspülmitteln verbesserte Varianten zur Verfügung, die entsprechend bevorzugt eingesetzt werden.

Die im Zusammenhang mit den eingesetzten Proteasen gemachten Angaben zu Mengen und Formulierungsformen treffen *mutatis mutandis* auch auf alle weiteren oben beschriebenen Enzyme zu.

Glaskorrosionsinhibitoren verhindern das Auftreten von Trübungen, Schlieren und Kratzern aber auch das Irisieren der Glasoberfläche von maschinell gereinigten Gläsern. Bevorzugte Glaskorrosionsinhibitoren stammen aus der Gruppe der Magnesium- und Zinksalze sowie der Magnesium- und Zinkkomplexe. Im Rahmen der vorliegenden Erfindung beträgt der Gehalt an Zinksalz in Geschirrspülmitteln, insbesondere maschinellen Geschirrspülmitteln vorzugsweise zwischen 0,1 bis 5 Gew.-%, bevorzugt zwischen 0,2 bis 4 Gew.-% und insbesondere zwischen 0,4 bis 3 Gew.-%, bzw. der Gehalt an Zink in oxidierter Form (berechnet als Zn₂+) zwischen 0,01 bis 1 Gew.-%, vorzugsweise zwischen 0,02 bis 0,5 Gew.-% und insbesondere zwischen 0,04 bis 0,2 Gew.-%, jeweils bezogen auf das Gesamtgewicht des Glaskorrosionsinhibitor-haltigen Mittels.

Um den Zerfall vorgefertigter Formkörper zu erleichtern, ist es möglich, Desintegrationshilfsmittel, so genannte Tablettensprengmittel, in diese Mittel einzuarbeiten, um die Zerfallszeiten zu verkürzen. Unter Tablettensprengmitteln bzw. Zerfallsbeschleunigern werden Hilfsstoffe verstanden, die für den raschen Zerfall von Tabletten in Wasser oder anderen Medien und für die zügige Freisetzung der Wirkstoffe sorgen. Bevorzugt können Desintegrationshilfsmittel in Mengen von 0,5 bis 10 Gew.-%, vorzugsweise 3 bis 7 Gew.-% und insbesondere 4 bis 6 Gew.-%, jeweils bezogen auf das Gesamtgewicht des desintegrationshilfsmittelhaltigen Mittels, eingesetzt werden.

Als Parfümöle bzw. Duftstoffe können im Rahmen der vorliegenden Erfindung einzelne Riechstoffverbindungen, z.B. die synthetischen Produkte vom Typ der Ester, Ether, Aldehyde, Ketone, Alkohole und Kohlenwasserstoffe verwendet werden. Bevorzugt werden jedoch Mischungen verschiedener Riechstoffe verwendet, die gemeinsam eine ansprechende Duftnote erzeugen. Solche Parfümöle können auch natürliche Riechstoffgemische enthalten, wie sie aus pflanzlichen Quellen zugänglich sind, z.B. Pinien-, Citrus-, Jasmin-, Patchouli-, Rosen- oder Ylang-Ylang-Öl.

Die Konfektionierung hierin beschriebener Geschirrspülmittel, insbesondere maschineller Geschirrspülmittel kann in unterschiedlicher Wiese erfolgen. Die Mittel können in fester oder flüssiger sowie als Kombination fester und flüssiger Angebotsformen vorliegen. Als feste Angebotsformen eignen sich insbesondere Pulver, Granulate, Extrudate, Kompaktate, insbesondere Tabletten. Die flüssigen Angebotsformen auf Basis von Wasser und/oder organischen Lösungsmitteln können verdickt, in Form von Gelen vorliegen. Hierin beschriebene Mittel können in Form einphasiger oder mehrphasiger Produkte konfektioniert werden.

Hierin beschriebene Geschirrspülmittel, insbesondere maschinelle Geschirrspülmittel werden vorzugsweise zu Dosiereinheiten vorkonfektioniert. Diese Dosiereinheiten umfassen vorzugsweise die für einen Reinigungsgang notwendige Menge an wasch- oder reinigungsaktiven Substanzen.

Die hierin beschriebenen Geschirrspülmittel, insbesondere maschinellen Geschirrspülmittel, insbesondere die vorgefertigten Dosiereinheiten weisen mit besonderem Vorzug eine wasserlösliche Umhüllung auf.

Die wasserlösliche Umhüllung wird vorzugsweise aus einem wasserlöslichen Folienmaterial, welches ausgewählt ist aus der Gruppe, bestehend aus Polymeren oder Polymergemischen, gebildet. Die Umhüllung kann aus einer oder aus zwei oder mehr Lagen aus dem wasserlöslichen Folienmaterial gebildet werden. Das wasserlösliche Folienmaterial der ersten Lage und der weiteren Lagen, falls vorhanden, kann gleich oder unterschiedlich sein. Besonders bevorzugt sind Folien, die beispielsweise zu Verpackungen wie Schläuchen oder Kissen verklebt und/oder versiegelt werden können, nachdem sie mit einem Mittel befüllt wurden.

Es ist bevorzugt, dass die wasserlösliche Umhüllung Polyvinylalkohol oder ein Polyvinylalkoholcopolymer enthält. Wasserlösliche Umhüllungen, die Polyvinylalkohol oder ein Polyvinylalkoholcopolymer enthalten, weisen eine gute Stabilität bei einer ausreichend hohen Wasserlöslichkeit, insbesondere Kaltwasserlöslichkeit, auf.

Geeignete wasserlösliche Folien zur Herstellung der wasserlöslichen Umhüllung basieren bevorzugt auf einem Polyvinylalkohol oder einem Polyvinylalkoholcopolymer, dessen Molekulargewicht im Bereich von 10.000 bis 1.000.000 gmol⁻¹, vorzugsweise von 20.000 bis 500.000 gmol⁻¹, besonders bevorzugt von 30.000 bis 100.000 gmol⁻¹ und insbesondere von 40.000 bis 80.000 gmol⁻¹ liegt.

Die Herstellung von Polyvinylalkohol geschieht üblicherweise durch Hydrolyse von Polyvinylacetat, da der direkte Syntheseweg nicht möglich ist. Ähnliches gilt für Polyvinylalkoholcopolymere, die aus entsprechend aus Polyvinylacetatcopolymeren hergestellt werden. Bevorzugt ist, wenn wenigstens eine Lage der wasserlöslichen Umhüllung einen Polyvinylalkohol umfasst, dessen Hydrolysegrad 70 bis 100 Mol-%, vorzugsweise 80 bis 90 Mol-%, besonders bevorzugt 81 bis 89 Mol-% und insbesondere 82 bis 88 Mol-% ausmacht.

Einem zur Herstellung der wasserlöslichen Umhüllung geeignetem Polyvinylalkohol-enthaltendem Folienmaterial kann zusätzlich ein Polymer ausgewählt aus der Gruppe umfassend (Meth)Acrylsäure-haltige (Co)Polymere, Polyacrylamide, Oxazolin-Polymere, Polystyrolsulfonate, Polyurethane, Polyester, Polyether, Polymilchsäure oder Mischungen der vorstehenden Polymere zugesetzt sein. Ein bevorzugtes zusätzliches Polymer sind Polymilchsäuren.

Bevorzugte Polyvinylalkoholcopolymere umfassen neben Vinylalkohol Dicarbonsäuren als weitere Monomere. Geeignete Dicarbonsäuren sind Itaconsäure, Malonsäure, Bernsteinsäure und Mischungen daraus, wobei Itaconsäure bevorzugt ist.

Ebenfalls bevorzugte Polyvinylalkoholcopolymere umfassen neben Vinylalkohol eine ethylenisch ungesättige Carbonsäure, deren Salz oder deren Ester. Besonders bevorzugt enthalten solche Polyvinylalkoholcopolymere neben Vinylalkohol Acrylsäure, Methacrylsäure, Acrylsäureester, Methacrylsäureester oder Mischungen daraus.

Es kann bevorzugt sein, dass das Folienmaterial weitere Zusatzstoffe enthält. Das Folienmaterial kann beispielsweise Weichmacher wie Dipropylenglycol, Ethylenglycol, Diethylenglycol, Propylenglycol, Glycerin, Sorbitol, Mannitol oder Mischungen daraus enthalten. Weitere Zusatzstoffe umfassen beispielsweise Freisetzungshilfen, Füllmittel, Vernetzungsmittel, Tenside, Antioxidationsmittel, UV-Absorber, Antiblockmittel, Antiklebemittel oder Mischungen daraus.

Geeignete wasserlösliche Folien zum Einsatz in den wasserlöslichen Umhüllungen der wasserlöslichen Verpackungen gemäß der Erfindung sind Folien, die von der Firma MonoSol LLC beispielsweise unter der Bezeichnung M8630, C8400 oder M8900 vertrieben werden. Andere geeignete Folien umfassen Folien mit der Bezeichnung Solublon^{®} PT, Solublon^{®} GA, Solublon^{®} KC oder Solublon^{®} KL von der Aicello Chemical Europe GmbH oder die Folien VF-HP von Kuraray.

Die entsprechende Verwendung der hierin beschriebenen Geschirrspülmittel ist ebenfalls Gegenstand der Erfindung. Ebenso betrifft die Erfindung ein Geschirrspülverfahren, insbesondere maschinelles Geschirrspülverfahren, bei welchem ein Geschirrspülmittel gemäß der Erfindung eingesetzt wird. Gegenstand der vorliegenden Anmeldung ist daher weiterhin ein Verfahren zur Reinigung von Geschirr in einer Geschirrspülmaschine, bei welchem das Geschirrspülmittel während des Durchlaufens eines Geschirrspülprogramms vor Beginn des Hauptspülgangs oder im Verlaufe des Hauptspülgangs in den Innenraum einer Geschirrspülmaschine eindosiert wird. Die Eindosierung bzw. der Eintrag des Geschirrspülmittels in den Innenraum der Geschirrspülmaschine kann manuell erfolgen, vorzugsweise wird das Mittel jedoch mittels der Dosierkammer in den Innenraum der Geschirrspülmaschine dosiert. In verschiedenen Ausführungsformen der Erfindung liegt bei solchen Geschirrspülverfahren die (Spül)Temperatur vorzugsweise bei 50 °C oder niedriger, besonders bevorzugt 45 °C oder niedriger, noch bevorzugter 40°C oder niedriger.

Eine typische Rahmenrezeptur für ein vorzugsweise einsetzbares maschinelles Geschirrspülmittel, beispielsweise in Tablettenform, umfasst folgende Stoffe:

| | |
|---|---|
| Na-Tripolyphosphat | 20-50 Gew.-% |
| Natriumcarbonat | 10-30 Gew.-% |
| Natriumpercarbonat | 5-18 Gew.-% |
| Bleichaktivator | 0,5-5 Gew.-% |
| Bleichkatalysator | 0,01-1 Gew.-% |
| Sulfopolymer | 2,5-15 Gew.-% |
| Polycarboxylat | 0,1-10 Gew.-% |
| Niotensid | 0,5-10 Gew.-% |
| Phosphonat | 0,5-5 Gew.-% |
| Proteasen | 0,1-5 Gew.-% |
| Amylase | 0,1-5 Gew.-%, |

wobei sich die Angabe in Gew.-% jeweils auf das gesamte Mittel beziehen. Statt des oder eines Teils des Tripolyphosphats kann in der Rezeptur insbesondere auch 10-50 Gew.-% Citrat oder MGDA oder GLDA oder EDDS oder Mischungen aus zwei oder drei dieser Substanzen eingesetzt werden.

### Beispiele

### Beispiel 1: Verbesserung der Proteaseleistung beim automatischen Geschirrspülen

In einer Bosch SMS86 Geschirrspülmaschine wurden bei 40 °C (Programm "Sanft 40") und 21°dH Porzellanteller mit einer Anschmutzung aus schwarzem Tee, Eigelb, Hackfleisch und Creme Brûlée mit einer festen Geschirrspülmitteltablette (20 g; Zusammensetzung siehe Tabelle 1) mit verschiedenen einzelnen Proteasen (V1-V5) oder Proteasekombinationen (M1-M3) gespült. Die Creme Brülee Anschmutzung wurde als hartnäckige Anschmutzung verwendet. Dazu wurde die fertige Mischung Creme Brûlée der Firma Debic in einem Topf auf 60°C erwärmt und jeweils 3,5 g mit einem Pinsel auf Dessertteller aufgetragen und für 2 h bei Raumtemperatur eintrocknen gelassen. Danach wurden die Teller in einen kalten Ofen (Binder) gestellt und innerhalb von 1 h auf 140°C erhitzt. Dann wurde die Creme Brûlée Anschmutzung für 2 h bei 140°C im Ofen eingebrannt.

Nach jedem Spülzyklus wurde die Reinigungsleistung visuell nach IKW bestimmt (Auswertung von 1-10, je höher der Wert, desto besser die Leistung, Unterschiede von mindestens 1 sind signifikant). Die Ergebnisse für die gestesteten Rezepturen sind in der Tabelle 2 als arithmetische Mittelwerte aufgelistet. Höhere Werte bedeuten eine bessere Reinigungsleistung.

**Tabelle 1: Zusammensetzung des maschinellen Geschirrspülmittels**

| | Basis |
|---|---|
| Phosphat (Gew.-%) | 35,9 |
| Natriumcarbonat (Gew.-%) | 12,2 |
| Phosphonat (Gew.-%) | 2,4 |
| Sulfonsäuregruppen-haltiges Polymer (Gew.-%) | 7,9 |
| Polyacrylat (Gew.-%) | 4,6 |
| Nichtionische Tenside (Gew.-%) | 6,1 |
| Percarbonat (Gew.-%) | 14,6 |
| TAED (Gew.-%) | 2,3 |
| Bleichkatalysator (Gew.-%) | 1,0 |
| Polycarboxylat (Gew.-%) | 1,5 |
| Natriumsilikat/Polycarboxylat (Gew.%) | 3,9 |
| Enzymzusammensetzung (Amylase) (Gew.-%) | 1,0 |
| Zinkacetat (Gew.-%) | 0,2 |
| Reste (Parfüm, Farbstoffe, Protease bzw. Proteasemischung etc.) (Gew.-%) | ad 100 |

Zur Formulierung gemäß Tabelle 1 wurde soviel der entsprechenden Proteasezubereitung (einzelne Proteasen, V1-V5) bzw. deren Mischungen (M1-M3) zugegeben, dass die Gesamtproteasemenge in der Formulierung 0,24 Gew.-% betrug.
V1: Basis + Enzym gemäß Seq ID No. 3
V2: Basis + Enzym gemäß Seq ID No. 4
V3: Basis + Enzym gemäß Seq ID No. 5
V4: Basis + Enzym gemäß Seq ID No. 6
V5: Basis + Savinase Ultra Plus 16L (Novozymes)
M1: Basis + Mischung zu gleichen Teilen bezogen auf den Proteasegehalt in Gew.-% aus Enzym gemäß Seq ID No. 3 und Enzym gemäß Seq ID No. 4 (erfindungsgemäß)
M2: Basis + Mischung zu gleichen Teilen bezogen auf den Proteasegehalt in Gew.-% aus Enzym gemäß Seq ID No. 3 und Enzym gemäß Seq ID No.5 (erfindungsgemäß)
M3: Basis + Mischung zu gleichen Teilen bezogen auf den Proteasegehalt in Gew.-% aus Enzym gemäß Seq ID No. 3 und Enzym gemäß Seq ID No.6 (nicht erfindungsgemäß)

**Tabelle 2: Reinigungsleistung**

| Protease/Proteasekombination | Tee | Hackfleisch | Eigelb | Crème Brûlée |
|---|---|---|---|---|
| V1 | 8,7 | 10,0 | 5,8 | 4,6 |
| V2 | 5,6 | 9,8 | 6,9 | 8,4 |
| V3 | 6,3 | 10,0 | 5,7 | 7,8 |
| V4 | 6,8 | 9,8 | 5,1 | 8,8 |
| V5 | 5,1 | 10,0 | 6,0 | 5,2 |
| M2 | 8,6 | 9,8 | 6,3 | 8,7 |
| | | | | |

| Protease/Proteasekombination | Tee | Hackfleisch | Eigelb | Crème Brûlée |
|---|---|---|---|---|
| V1 | 9,5 | 9,7 | 6,3 | 4,1 |
| M1 | 8,7 | 9,5 | 6,1 | 6,8 |
| M3 | 9,3 | 9,7 | 6,2 | 7,6 |

Wie man der Tabelle 2 entnehmen kann zeigt die Protease gemäß Seq ID No 3 (V1) eine sehr gute Reinigungsleistung an Teeanschmutzungen, während die Reinigungsleistung an Creme Brûlée nur mittelmäßig ist. Dagegen erkennt man bei den Proteasen gemäß Seq ID No 4, 5 und 6 (V2 bis V4) eine gute Leistung an Creme Brûlée, während die Leistung an Tee nur mittelmäßig ist. Savinase (V5) zeigt weder an Tee noch an Creme Brûlée eine gute Leistung.

Wie man bei allen getesteten Proteasekombinationen sieht, ist sowohl die Teereinigungsleistung als auch die Reinigungsleistung an Creme Brûlée auf einem hohen Niveau. Der Tabelle 2 ist eindeutig zu entnehmen, dass die Kombination von zwei verschiedenen Proteasen zu einer deutlichen Verbesserung der Reinigungsleistung führt (M1 bis M3).

## Patentansprüche

1. Reinigungsmittel, **dadurch gekennzeichnet, dass**
es mindestens eine erste und eine zweite Protease umfasst, wobei
a) die erste Protease eine Aminosäuresequenz gemäß SEQ ID NO. 3 aufweist, und
b) die zweite Protease ausgewählt ist aus der Gruppe gebildet aus
b1) Protease, die eine Aminosäuresequenz umfasst, die zu der in SEQ ID NO. 2 angegebenen Aminosäuresequenz über deren Gesamtlänge zu mindestens 95% identisch ist und in der Zählung gemäß SEQ ID NO. 2 an Position 99 die Aminosäure Glutaminsäure (E) aufweist;
b2) Protease, die eine Aminosäuresequenz umfasst, die zu der in SEQ ID NO. 2 angegebenen Aminosäuresequenz über deren Gesamtlänge zu mindestens 95% identisch ist und in der Zählung gemäß SEQ ID NO. 2 die Aminosäuresubstitution R99E in Kombination mit mindestens zwei weiteren Aminosäuresubstitutionen aufweist, die ausgewählt sind aus der Gruppe bestehend aus S3T, V4I und V199I; und
das Reinigungsmittel ein Geschirrspülmittel, insbesondere ein maschinelles Geschirrspülmittel ist,
die Protease gemäß b1) eine Aminosäuresequenz aufweist, die in der Zählung gemäß SEQ ID NO. 2 in den Positionen 1-98 und 100-269 SEQ ID NO. 2 entspricht und an Position 99 die Aminosäure Glutaminsäure (E) aufweist; und
die Protease b2) eine Aminosäuresequenz gemäß SEQ ID NO. 2 mit den Aminosäuresubstitutionen R99E, S3T, V4I und V199I in der Zählung gemäß SEQ ID NO. 2 aufweist.

2. Reinigungsmittel nach Anspruch 1, **dadurch gekennzeichnet, dass** es eine erste Protease und eine zweite Protease in einem Massenverhältnis von 10:1 bis 1:10 enthält.

3. Reinigungsmittel nach einem der Ansprüche 1 bis 2, **dadurch gekennzeichnet, dass** der Gewichtsanteil jeder der Proteasen, bezogen auf das entsprechende aktive Protein, am Gesamtgewicht des Mittels von 1 × 10-8 bis 5 Gew.-%, bevorzugt 0,001 bis 2 Gew.-%, weiter bevorzugt von 0,005 bis 1,5 Gew.-%, noch weiter bevorzugt von 0,01 bis 1 Gew.-% und besonders bevorzugt von 0,01 bis 0,5 Gew.-%, beträgt.

4. Reinigungsmittel nach einem der Ansprüche 1 bis 3, **dadurch gekennzeichnet, dass** es mindestens einen weiteren Bestandteil, vorzugsweise mindestens zwei weitere Bestandteile, ausgewählt aus der Gruppe bestehend aus Gerüststoffen, Tensiden, Polymeren, Bleichmitteln, Bleichkatalysatoren, Bleichaktivatoren, Nicht-Protease-Enzymen, Korrosionsinhibitoren, Glaskorrosionsinhibitoren, Desintegrationshilfsmitteln, Duftstoffen und Parfümträgern enthält.

5. Reinigungsmittel nach einem der Ansprüche 1 bis 4, **dadurch gekennzeichnet, dass** das
(1) maschinelle Geschirrspülmittel in fester Form vorliegt; und/oder
(2) maschinelle Geschirrspülmittel in vorportionierter Form vorliegt; und/oder
(3) maschinelle Geschirrspülmittel mehrere räumlich voneinander getrennte Zusammensetzungen aufweist.

6. Verwendung eines Reinigungsmittels nach einem der Ansprüche 1 bis 5 in einem maschinellen Geschirrspülverfahren.

7. Verfahren zur Reinigung von Geschirr in einer automatischen Geschirrspülmaschine, **dadurch gekennzeichnet, dass** ein Reinigungsmittel nach einem der Ansprüche 1 bis 6 während des Durchlaufens eines Geschirrspülprogramms vor Beginn des Hauptspülgangs oder im Verlaufe des Hauptspülgangs in den Innenraum einer Geschirrspülmaschine eindosiert wird.

8. Verwendung einer Kombination einer ersten und einer zweiten Protease zur Verbesserung der Reinigungsleistung, insbesondere der Reinigungsleistung an enzymsensitiven Anschmutzungen, eines Geschirrspülmittels, vorzugsweise eines maschinellen Geschirrspülmittels, **dadurch gekennzeichnet, dass**
a) die erste Protease eine Aminosäuresequenz gemäß SEQ ID NO. 3 aufweist, und
b) die zweite Protease ausgewählt ist aus der Gruppe gebildet aus
b1) Protease, die eine Aminosäuresequenz umfasst, die zu der in SEQ ID NO. 2 angegebenen Aminosäuresequenz über deren Gesamtlänge zu mindestens 95% identisch ist und in der Zählung gemäß SEQ ID NO. 2 an Position 99 die Aminosäure Glutaminsäure (E) aufweist; und
b2) Protease, die eine Aminosäuresequenz umfasst, die zu der in SEQ ID NO. 2 angegebenen Aminosäuresequenz über deren Gesamtlänge zu mindestens 95% identisch ist und in der Zählung gemäß SEQ ID NO. 2 die Aminosäuresubstitution R99E in Kombination mit mindestens zwei weiteren Aminosäuresubstitutionen aufweist, die ausgewählt sind aus der Gruppe bestehend aus S3T, V4I und V199I;
wobei
die Protease gemäß b1) eine Aminosäuresequenz aufweist, die in der Zählung gemäß SEQ ID NO. 2 in den Positionen 1-98 und 100-269 SEQ ID NO. 2 entspricht und an Position 99 die Aminosäure Glutaminsäure (E) aufweist; und
die Protease b2) eine Aminosäuresequenz gemäß SEQ ID NO. 2 mit den Aminosäuresubstitutionen R99E, S3T, V4I und V199I in der Zählung gemäß SEQ ID NO. 2 aufweist.

## Claims

1. A cleaning agent, **characterized in that**
it comprises at least a first and a second protease,
a) the first protease having an amino acid sequence according to SEQ ID NO. 3, and
b) the second protease being selected from the group formed by
b1) protease having an amino acid sequence which is at least 95% identical to the amino acid sequence indicated in SEQ ID NO. 2 over its entire length and has the amino acid glutamic acid (E) at position 99 using the numbering according to SEQ ID NO. 2;
b2) protease having an amino acid sequence which is at least 95% identical to the amino acid sequence indicated in SEQ ID NO. 2 over its entire length and has the amino acid substitution R99E using the numbering according to SEQ ID NO. 2 in combination with at least two further amino acid substitutions selected from the group consisting of S3T, V4I and V199I; and
the cleaning agent being a dishwasher detergent, in particular an automatic dishwasher detergent,
the protease according to b1) having an amino acid sequence which corresponds to SEQ ID NO. 2 in positions 1-98 and 100-269 and having the amino acid glutamic acid (E) at position 99 using the numbering according to SEQ ID NO. 2; and
the protease b2) having an amino acid sequence according to SEQ ID NO. 2 with the amino acid substitutions R99E, S3T, V4I and V199I using the numbering according to SEQ ID NO. 2.

2. The cleaning agent according to claim 1, **characterized in that** it contains a first protease and a second protease in a mass ratio of from 10:1 to 1:10.

3. The cleaning agent according to one of claims 1 to 2,
**characterized in that** the proportion by weight of each of the proteases, based on the corresponding active protein, with respect to the total weight of the agent is from 1 × 10-8 to 5 wt.%, preferably from 0.001 to 2 wt.%, more preferably from 0.005 to 1.5 wt.%, even more preferably from 0.01 to 1 wt.% and particularly preferably from 0.01 to 0.5 wt.%.

4. The cleaning agent according to one of claims 1 to 3,
**characterized in that** it contains at least one further component, preferably at least two further components, selected from the group consisting of builders, surfactants, polymers, bleaching agents, bleach catalysts, bleach activators, nonprotease enzymes, corrosion inhibitors, glass corrosion inhibitors, disintegration auxiliaries, fragrances and perfume carriers.

5. The cleaning agent according to one of claims 1 to 4,
**characterized in that**
(1) the automatic dishwasher detergent is in solid form; and/or
(2) the automatic dishwasher detergent is in a pre-portioned form; and/or
(3) the automatic dishwasher detergent has a plurality of compositions that are spatially separated from one another.

6. The use of a cleaning agent according to one of claims 1 to 5 in an automatic dishwashing method.

7. A method for cleaning dishes in an automatic dishwasher, **characterized in that** a cleaning agent according to one of claims 1 to 6 is metered into the interior of a dishwasher while a dishwashing program is running, before the main washing cycle begins or during the main washing cycle.

8. The use of a combination of a first and a second protease for improving cleaning performance, in particular the cleaning performance with respect to enzyme-sensitive soiling, of a cleaning agent, preferably an automatic dishwasher detergent, **characterized in that**
a) the first protease has an amino acid sequence according to SEQ ID NO. 3, and
b) the second protease is selected from the group formed by
b1) protease having an amino acid sequence which is at least 95% identical to the amino acid sequence indicated in SEQ ID NO. 2 over its entire length and has the amino acid glutamic acid (E) at position 99 using the numbering according to SEQ ID NO. 2; and
b2) protease having an amino acid sequence which is at least 95% identical to the amino acid sequence indicated in SEQ ID NO. 2 over its entire length and has the amino acid substitution R99E using the numbering according to SEQ ID NO. 2 in combination with at least two further amino acid substitutions selected from the group consisting of S3T, V4I and V199I;
the protease according to b1) having an amino acid sequence which corresponds to SEQ ID NO. 2 in positions 1-98 and 100-269 and having the amino acid glutamic acid (E) at position 99 using the numbering according to SEQ ID NO. 2; and
the protease b2) having an amino acid sequence according to SEQ ID NO. 2 with the amino acid substitutions R99E, S3T, V4I and V199I using the numbering according to SEQ ID NO. 2.

## Revendications

1. Agent de nettoyage, **caractérisé en ce que**
il comprend au moins une première et une seconde protéase, dans lequel
a) la première protéase présente une séquence d'acides aminés selon SEQ ID NO : 3, et
b) la seconde protéase est choisie dans le groupe constitué de
b1) protéase qui comprend une séquence d'acides aminés qui est identique à au moins 95 % à la séquence d'acides aminés indiquée dans SEQ ID NO : 2 sur toute sa longueur et qui présente l'acide aminé acide glutamique (E) en position 99 dans le comptage selon SEQ ID NO : 2 ;
b2) protéase qui comprend une séquence d'acides aminés qui est identique à au moins 95 % à la séquence d'acides aminés indiquée dans SEQ ID NO : 2 sur toute sa longueur et qui présente la substitution d'acide aminé R99E en combinaison avec au moins deux autres substitutions d'acides aminés choisies dans le groupe constitué de S3T, V4I et V199I dans le comptage selon SEQ ID NO : 2 ; et
l'agent de nettoyage est un agent de lavage pour vaisselle, en particulier un agent de lavage pour lave-vaisselle automatique,
la protéase selon b1) présente une séquence d'acides aminés qui correspond dans le comptage selon SEQ ID NO : 2 aux positions 1 à 98 et 100 à 269 de SEQ ID NO : 2 et présente l'acide aminé acide glutamique (E) en position 99 ; et
la protéase b2) présente une séquence d'acides aminés selon SEQ ID NO : 2 comportant les substitutions d'acides aminés R99E, S3T, V4I et V199I dans le comptage selon SEQ ID NO : 2.

2. Agent de nettoyage selon la revendication 1, **caractérisé en ce qu'**il contient une première protéase et une seconde protéase dans un rapport massique allant de 10:1 à 1:10.

3. Agent de nettoyage selon l'une des revendications 1 à 2,
**caractérisé en ce que** la proportion en poids de chacune des protéases, par rapport à la protéine active correspondante, par rapport au poids total de l'agent, va de 1 × 10 - 8 à 5 % en poids, de préférence de 0,001 à 2 % en poids, de manière particulièrement préférée de 0,005 à 1,5 % en poids, de manière très particulièrement préférée de 0,01 à 1 % en poids et de manière extrêmement préférée de 0,01 à 0,5 % en poids.

4. Agent de nettoyage selon l'une des revendications 1 à 3,
**caractérisé en ce qu'**il contient au moins un autre constituant, préférentiellement au moins deux autres constituants, choisis dans le groupe constitué d'adjuvants, tensioactifs, polymères, agents de blanchiment, catalyseurs de blanchiment, activateurs de blanchiment, enzymes non protéases, inhibiteurs de corrosion, inhibiteurs de corrosion du verre, auxiliaires de désintégration, parfums et supports de parfum.

5. Agent de nettoyage selon l'une des revendications 1 à 4,
**caractérisé en ce que**
(1) l'agent de lavage pour lave-vaisselle automatique se présente sous forme solide ; et/ou
(2) l'agent de lavage pour lave-vaisselle automatique se présente sous forme préportionnée ; et/ou
(3) l'agent de lavage pour lave-vaisselle automatique présente plusieurs compositions séparées physiquement les unes des autres.

6. Utilisation d'un agent de nettoyage selon l'une des revendications 1 à 5 dans un procédé de lavage de lave-vaisselle automatique.

7. Procédé pour le nettoyage de vaisselle dans un lave-vaisselle automatique, **caractérisé en ce qu'**un agent de nettoyage selon l'une des revendications 1 à 6 est dosé dans l'espace intérieur d'un lave-vaisselle automatique pendant le fonctionnement d'un programme de lave-vaisselle avant le début du cycle de lavage principal ou au cours du cycle de lavage principal.

8. Utilisation d'une combinaison d'une première et d'une seconde protéase pour l'amélioration du pouvoir nettoyant, en particulier du pouvoir nettoyant sur les salissures sensibles aux enzymes, d'un agent de lavage pour vaisselle, préférentiellement d'un agent de lavage pour lave-vaisselle automatique, **caractérisée en ce que**
a) la première protéase présente une séquence d'acides aminés selon SEQ ID NO : 3, et
b) la seconde protéase est choisie dans le groupe constitué de
b1) protéase qui comprend une séquence d'acides aminés qui est identique à au moins 95 % à la séquence d'acides aminés indiquée dans SEQ ID NO : 2 sur toute sa longueur et qui présente l'acide aminé acide glutamique (E) en position 99 dans le comptage selon SEQ ID NO : 2 ; et
b2) protéase qui comprend une séquence d'acides aminés qui est identique à au moins 95 % à la séquence d'acides aminés indiquée dans SEQ ID NO : 2 sur toute sa longueur et qui présente la substitution d'acide aminé R99E en combinaison avec au moins deux autres substitutions d'acides aminés choisies dans le groupe constitué de S3T, V4I et V199I dans le comptage selon SEQ ID NO : 2 ;
dans laquelle
la protéase selon b1) présente une séquence d'acides aminés qui correspond dans le comptage selon SEQ ID NO : 2 aux positions 1 à 98 et 100 à 269 de SEQ ID NO : 2 et présente l'acide aminé acide glutamique (E) en position 99 ; et
la protéase b2) présente une séquence d'acides aminés selon SEQ ID NO : 2 comportant les substitutions d'acides aminés R99E, S3T, V4I et V199I dans le comptage selon SEQ ID NO : 2.
